# EUROPEAN PATENT SPECIFICATION

(11) **EP 2 120 936 B1**
(45) Date of publication and mention of the grant of the patent: **22.06.2011**
(21) Application number: 08707469.6
(22) Date of filing: 31.01.2008
(51) Int. Cl.: A61K 31/439, A61P 11/06, A61P 11/08

(54) **NOVEL METHODS**
NEUE VERFAHREN
NOUVELLES MÉTHODES

(30) Priority: 21.02.2007 US 902843 P
(43) Date of publication of application: 25.11.2009
(73) Proprietor: Almirall, S.A., 08022 Barcelona (ES)
(72) Inventor: BELETA SUPERVIA, Jorge, E-08173 Sant Cugat del Valles (ES)
(74) Representative: Srinivasan, Ravi Chandran
(86) International application number: PCT/EP2008/000782
(87) International publication number: WO 2008/101591

(56) References cited:
- WO-A-2005/097126
- WO-A-2005/115462
- MOLFINO N A: "DRUGS IN CLINICAL DEVELOPMENT FOR CHRONIC OBSTRUCTIVE PULMONARY DISEASE" RESPIRATION, KARGER, BASEL, CH, vol. 72, no. 1, 2005, pages 105-112, XP008050164 ISSN: 0025-7931
- ALABASTER V A: "DISCOVERY & DEVELOPMENT OF SELECTIVE M3 ANTAGONISTS FOR CLINICAL USE" LIFE SCIENCES, PERGAMON PRESS, OXFORD, GB, vol. 60, no. 13/14, 1997, pages 1053-1060, XP001056370 ISSN: 0024-3205

## Description

### Field of the Invention

The invention relates to novel methods of anticholinergic therapy, particularly for respiratory diseases such as asthma and chronic obstructive pulmonary disease (COPD), without causing the class-related adverse effects of antimuscarinic compounds.

### Background of the Invention

Aclidinium (3(R)-(2-hydroxy-2,2-dithien-2-ylacetoxy)-1-(3-phenoxypropyl)-1-azoniabicyclo[2.2.2] octane) is a potent muscarinic receptor antagonist described, e.g., in WO 01/04118, WO 05/115467, WO 05/115466, and WO 05/115462 the contents of which applications are incorporated herein by reference. Aclidinium is a long-acting bronchodilator intended for administration by inhalation for treatment of respiratory diseases, especially asthma and COPD), currently in clinical trials.

Currently available muscarinic receptor antagonists include tiotropium ((1α,2β,4β,7β)-7-[(2-hydroxy-2,2-dithienylacetoxy]-9,9-dimethyl-3-oxa-9-azoniatricyclo[3.3.1.0^{2,4}]nonane), ipratropium ([8-methyl-8-(1-methylethyl)-8-azoniabicyclo[3.2.1]oct-3-yl]3-hydroxy-2-phenyl-propanoate), and glycopyrrolate ((1,1-dimethyl-2,3,4,5-tetrahydropyrrol-3-yl) 2-cyclopentyl-2-hydroxy-2-phenyl-acetate).

Acetylcholine is a neurotransmitter associated with parasympathetic innervation in the body and also with transmissions in the brain. It helps control the functioning of the heart, blood vessels, airways, and organs of the urinary and digestive tracts. It is also involved in memory, learning, and concentration. Antimuscarinic compounds inhibit the effects of acetylcholine on muscarinic receptors, which are by far the most common type of cholinergic receptors in the body. Compounds that inhibit acetylcholine activity at the M3 muscarinic receptors in the airways are very useful in the treatment of respiratory diseases, as they inhibit the acetylcholine-mediated contraction of smooth muscle in the airways, resulting in bronchodilation, and also reduce mucus secretion in the lungs.

One problem with the use of antimuscarinic compounds in the treatment of respiratory diseases, however, is the risk of side effects related to systemic suppression of cholinergic activity. These can include, for example, dry mouth, throat irritation, decreased sweating, increased pupil size, blurred vision, increased intraocular pressure, increased heart rate, chest pain, decreased gastric motility, constipation difficulty starting and continuing to urinate, and loss of bladder control due to overflow incontinence. Anticholinergic activity can also have effects on the central nervous system, such as impaired concentration, confusion, agitation, anxiety, delirium, attention deficit, impaired memory, light-headedness, drowsiness, and respiratory depression. It has been found that cholinesterase inhibitors, which inhibit the breakdown of acetylcholine, are beneficial in Alzheimer's disease and dementia, thus a physician may wish to avoid anticholinergic drugs in such patients if feasible. Acetylcholine has a complicated role in Parkinson's disease patients. It is believed to have a role in facilitating dopamine release, possibly through actions at the M4 and M5 muscarinic receptors in the brain, and on this basis, cholinesterase inhibitors are sometimes prescribed for Parkinson's patients; yet especially before the advent of levodopa, anticholinergics were used to treat the symptoms of Parkinson's disease, possibly by blocking the dopamine inhibiting activity of the M1 muscarinic receptors.

Older patients are more likely to experience undesired anticholinergic effects because their bodies produce less acetylcholine. Also, cells in many parts of the body (such as the digestive tract) in older patients may have fewer acetylcholine receptors. Thus, the acetylcholine produced is less likely to have an effect, and the effect of anticholinergic drugs is correspondingly greater. Moreover, older patients may have reduced kidney and/or liver function, and so may be prone to increased serum concentrations of many anticholinergic drugs,. As discussed below, a number of commonly prescribed medications have anticholinergic effects, so patients who are taking multiple medications with anticholinegic side effects may be at elevated risk. Older men in particular may suffer adverse effects, because the urinary difficulties associated with anticholinergic activity may exacerbate or be exacerbated by an enlarged or obstructed prostate. Overall, anticholinergic side effects are among the most common drug-related negative effects experienced by elderly people.

Currently marketed antimuscarinics may be unsuitable for use in patients having a susceptibility to conditions that may be exacerbated by systemic anticholinergic effects. Levels of systemic anticholinergic activity that may be easily tolerated in a young, healthy person may be unacceptable in such patients. Conditions that may be exacerbated by systemic anticholinergic effects include schizophrenia, glaucoma, dry eyes, enlarged or obstructed prostate, narrowing or obstructon of the small intestine, enlarged colon, chronic constipation, enlarged lower esophagus, heart disease (especially any condition that may be aggravated by tachycardia, for example restenosis or plaque in the coronary arteries, propensity to arrhythmias, damage resulting from prior heart attacks, and congestive heart failure), Parkinson's disease, Alzheimer's disease, dementia, and myasthenia gravis. Antimuscarinics may also present special risks when co-administered with drugs which have anticholinergic effects, for example atypical antipsychotics or tricyclic antidepressants. Antihistamines, particularly first generation sedating antihistamines such as diphenhydramine, may bind muscarinic receptors in addition to histamine type-1 receptors, and so may also have anticholinergic effects. In extreme cases, anticholinergic drugs can trigger anticholinergic delerium, a medical emergency characterized by hot, dry skin, dry mucus membranes, dilated pupils, absent bowel sounds, and tachycardia. Finally, systemically active antimuscarinics may interfere with the action of drugs intended to enhance acetylcholine function, for example cholinesterase inhibitors and cholinergic agonists.

Accordingly, there is a need for antimuscarinic therapy, particularly for respiratory diseases, especially asthma and chronic obstructive pulmonary disease (COPD), which does not cause the class-related adverse effects of systemically active antimuscarinic compounds.
WO 2005/115462 relates to a combination which comprises (a) a PDE4 inhibitor and (b) an antagonist of M3 muscarinic receptors which is 3(R)-(2-hydroxy-2,2-dithien-2-ylacetoxy)-1(3-phenoxypropyl)-1-azoniabicyclo[2.2.2]oct ane, in the form of a salt having an anion X, which is a pharmaceutically acceptable anion of a mono or polyvalent acid
WO 2005/097126 relates to the use of anticholinergic agents for producing a medicament for the prophylaxis and treatment of proliferative processes.
Molfino N A, Respiration, 2005, 72, 105-112 relates to the use of selective M3 antagonists in the treatment of COPD and asthma.

### Summary of the Invention

It has now been discovered that aclidinium may be used in the treatment of respiratory diseases without exposing patients to the class-related adverse effects of systemically active antimuscarinic compounds. Although aclidinium has the same ester moiety as, e.g., tiotropium (2-hydroxy-2,2-dithien-2-ylacetoxy), aclidinium administered by inhalation is surprisingly much more subject to degradation in plasma to its inactive acid and alcohol metabolites. Consequently, systemic exposure to the compound is negligible. Because of aclidinium's rapid metabolization, it is unlikely to result in undesirable systemic anticholinergic effects. Aclidinium nevertheless has a long duration of action at the receptor and is capable of providing long-acting benefits of antimuscarinic therapy to lungs and airways.
Accordingly, the invention provides, in a first embodiment, aclidinium, for use in the treatment or prevention of a respiratory disease or condition in a patient by inhalation, which patient is suffering from or susceptible to a condition which may be exacerbated by systemic antimuscarinic activity.
Typically, the respiratory disease is a disease that may be treated, ameliorated or inhibited by a muscarinic receptor antagonist. More preferably, the respiratory disease or condition is selected from acute or chronic bronchitis, emphysema, asthma and chronic obstructive pulmonary disease, especially asthma and chronic obstructive pulmonary disease, most especially chronic obstructive pulmonary disease.
Typically, the patient is suffering from or susceptible to one or more conditions selected from
a. schizophrenia, impaired concentration, confusion, agitation, delirium, attention deficit, impaired memory, respiratory depression.
b. glaucoma, dry eye, increased pupil size, blurred vision, increased intraocular pressure,
c. enlarged or obstructed prostate, difficulty urinating, overflow incontinence,
d. narrowing or obstruction of the small intestine, enlarged colon, chronic constipation, enlarged lower esophagus, decreased gastric motility, constipation,
e. dry mouth, throat irritation, impaired sweating
f. cardiovascular disease (including any of restenosis, arteriosclerosis, prior stroke or heart attack, congestive heart failure), arrhythmia, tachycardia,
g. Parkinson's disease, Alzheimer's disease, dementia, and/or
h. myasthenia gravis
Typically, the patient is a male. Further, the patient is typically over sixty years old.
In a further embodiment of the invention, the medicament is for administration to a patient who intends to drive or operate machinery during the course of treatment.
In a further embodiment of the invention, the patient is receiving a second drug which is a systemically active anticholinergic agent, or an agent which may cause or exacerbate any of the conditions listed above. Typically, the second drug is selected from antipsychotics, tricyclic antidepressants, and antihistamines.
In a further embodiment of the invention, the patient is receiving a drug which is intended to enhance acetylcholine function, e.g., a cholinesterase inhibitor or cholinergic agonist, e.g., as set forth below.
Typically, the aclidinium is in the form of a salt with an anion X, wherein X is a pharmaceutically acceptable anion of a mono or polyvalent acid. More typically, X is an anion derived from an inorganic acid, such as hydrochloric acid, hydrobromic acid, sulphuric acid and phosphoric acid, or an organic acid such as methanesulphonic acid, acetic acid, fumaric acid, succinic acid, lactic acid, citric acid and maleic acid. Preferably, the aclidinium is in the form of aclidinium bromide.
Typically, the aclidinium is in the form of a dry powder suitable for inhalation.
Typically, the medicament comprises a pharmaceutically acceptable carrier selected from mono-, di- or polysaccharides and sugar alcohols. Preferably, the carrier is lactose.
Typically, the patient receives one or more additional medication for treatment of the respiratory disease or condition. More typically, the additional medication for treatment of the respiratory disease or condition is selected from beta-adrenergic agonists, corticosteroids or glucocorticoids, PDE IV inhibitors, antihistamines, anti-IgE antibodies, leukotriene D4 inhibitors, inhibitors of egfr-kinase, p38 kinase inhibitors and/or NK1-receptor antagonists; e.g., selected from the compounds identified below. Preferably, the additional medication is selected from corticosteroids and/or beta-adrenergic agonists.

The invention further provides use of aclidinium, as defined above, in the manufacture of a medicament for use in the treatment or prevention, by inhalation, of a respiratory disease or condition, as defined above.

The invention further provides a pharmaceutical composition for use in the treatment or prevention, by inhalation, of a respiratory disease or condition as defined above in a patient as defined above, which pharmaceutical composition comprises a pharmaceutically acceptable carrier, and, as active principle aclidinium, as defined above.

### Detailed description of the invention

Medications which may have anticholinergic effects or make patients more susceptible to anticholinergic effects, include, for example,
a. Drugs for nausea or dizziness, especially anticholinergic agents, e.g., promethazine (Phenergan), prochlorperazine (Compazine), trimethobenzamide (Tigan), meclizine (Antivert), cyclizine (Marezine), scopalamine
b. Drugs for Parkinson's Disease, especially anticholinergic agents, e.g., benztropine; biperiden; procyclidine; trihexyphenidyl; ethoproprazine
c. Antidepressants, especially tricyclics, e.g., amitriptyline (Elavil), doxepin (Sinequan), imipramine (Tofranil), trimipramine (Surmontil), nortriptyline (Pametor), protriptyline (Vivactil). amoxapine (Asendin), maprotiline (Ludiomil), clomipramine (Anafranil); desipramine (Norpramin)
d. Antihistamines, especially first-generation sedating antihistamines, e.g., diphenhydramine (Benadryl) chlorpheniramine (Chlor-Trimeton), hydroxyzine (Atarax/Vistaril), cyproheptadine (Periactin)
e. Muscle relaxants, e.g., metaxalone (Skelaxin) cyclobenzaprine (flexeril), orphenadrine (Norflex)
f. Certain anti-migrane medications, e.g., belladonna alkaloids
g. Certain anti-diarrhea drugs, e.g., diphenoxylate/atropine (Lomotil)
h. Urinary and GI Antispasmodics, e.g., oxybutynin (Ditropan), flavoxate (Urispas), dicyclomine (Bentyl), hyoscyamine; belladonna alkaloids; tolterodine (Detrol), trospium, clindinium; propantheline, pirenzepine, telenzepine,
i. Antiarrhythmic drugs, e.g., disopyramide (Norpace), procainamide (Pronestyl), quinidine, atropine
j. Antipsychotics, e.g., chlorpromazine (Thorazine), thioridazine (Mellaril), clozapine (Clozaril), fluphenazine (Stelazine), thiothixene (Navane)

Medications which enhance cholinergic activity, include
a. Reversible cholinesterase inhibitors, e.g., edrophonium, tacrine, donepizil, physostigmine, pyridostigmine, rivastigmine, galantamine, neostigmine,
b. Cholinergic agonists, e.g., methacholine, bethanachol, pilocarpine

Beta-adrenergic agonists that can be combined with aclidinium in the present invention particularly include β2 adrenergic agonists useful for treatment of respiratory diseases or conditions, for example, selected from the group consisting of arformoterol, bambuterol, bitolterol, broxaterol, carbuterol, clenbuterol, dopexamine, fenoterol, formoterol, hexoprenaline, ibuterol, isoprenaline, mabuterol, meluadrine, nolomirole, orciprenaline, pirbuterol, procaterol, reproterol, ritodrine, rimoterol, salbutamol, salmeterol, sibenadel, sulfonterol, terbutaline, tulobuterol, GSK-597901,GSK-159797,KUL-1248,TA-2005 and QAB-1491, in free or pharmaceuticaly acceptable salt form. Preferably, the β2 adrenergic agonist is a long-acting β2 adrenergic agonist, e.g., selected from the group consisting of formoterol, salmeterol and QAB-149 in free or pharmaceutically acceptable salt form.

Corticosteroids that can be combined with aclidinium in the present invention particularly include those suitable for administration by inhalation in the treatment of respiratory diseases or conditions, e.g., prednisolone, methylprednisolone, dexamethasone, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, deprodone propionate, fluticasone propionate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, hydrocortisone acetate, hydrocortisone sodium succinate, prednisolone sodium phosphate and hydrocortisone probutate. Budesonide and mometasone are especially preferred.

PDE4 inhibitors that can be combined with aclidinium in the present invention include denbufylline, rolipram, cipamfylline, arofylline, filaminast, piclamilast, mesopram, drotaverine hydrochloride, lirimilast, roflumilast, cilomilast, 6-[2-(3,4-Diethoxyphenyl)thiazol-4-yl]pyridine-2-carboxylic acid, (R)-(+)-4-[2-(3-Cyclopentyloxy-4-methoxyphenyl)-2-phenylethyl]pyridine, N-(3,5-Dichloro-4-pyridinyl)-2-[1-(4-fluorobenzyl)-5-hydroxy-1H-indol-3-yl]-2-oxacetamide, 9-(2-Fluorobenzyl)-N6-methyl-2-(trifluoromethyl)adenine, N-(3,5-Dichloro-4-pyridinyl)-8-methoxyquinoline-5-carboxamide, N-[9-Methyl-4-oxo-1-phenyl-3,4,6,7-tetrahydropyrrolo[3,2,1-jk][1,4]benzodiazepin-3(R)-yl]pyridine-4-carboxamide, 3-[3-(Cyclopentyloxy)-4-methoxybenzyl]-6-(ethylamino)-8-isopropyl-3H-purine hydrochloride, 4-[6,7-Diethoxy-2,3-bis(hydroxymethyl)naphthalen-1-yl]-1-(2, methoxyethyl)pyridin-2(1H)-one, 2-carbomethoxy-4-cyano-4-(3-cyclopropylmethoxy-4-difluroromelhoxyphenyl)cylohexan1-one, cis [4-cyano-4-(3-cyclopropylmethoxy-4-difluoromethoxyphenyl)cyclohexan-1-ol, ONO-6126 (Eur Respir J 2003, 22(Suppl. 45): Abst 2557) and the compounds claimed in the PCT patent applications number WO03/09761 and PCT/EP03/14722 and in the Spanish patent application number P200302613.

LTD4 antagonists that can be combined with aclidinium in the present invention include tomelukast, Ibudilast, pobilukast, pranlukast hydrate, zafirlukast, ritolukast, verlukast, sulukast, cinalukast, iralukast sodium, montelukast sodium, 4-[4-[3-(4-Acetyl-3-hydroxy-2-propylphenoxy)propylsulfonyl]phenyl]-4-oxobutyric acid, [[5-[[3-(4-Acetyl-3-hydroxy-2-propylphenoxy)propyl]thio]-1,3,4-thiadiazol-2-yl]thio]acetic acid, 9-[(4-Acetyl-3-hydroxy-2-n-propylphenoxy)methyl]-3-(1H-tetrazol-5-yl)-4H-pyrido[1,2-a]pyrimidin-4-one, 5-[3-[2-(7-Chloroquinolin-2-yl)vinyl]phenyl]-8-(N,N-dimethylcarbamoyl)-4,6-dithiaoctanoic acid sodium salt; 3-[1-[3-[2-(7-Chloroquinolin-2-yl)vinyl]phenyl]-1-[3-(dimethylamino)-3-oxopropylsulfanyl]methylsulfanyl]propionic acid sodium salt, 6-(2-Cyclohexylethyl)-[1,3,4]thiadiazolo[3,2-a]-1,2,3-triazolo[4,5-d]pyrimidin-9(1)-one, 4-[6-Acetyl-3-[3-(4-acetyl-3-hydroxy-2-propylphenylthio)propoxy]-2-propylphenoxy]butyric acid, (R)-3-Methoxy-4-[1-methyl-5-[N-(2-methyl-4,4,4-trifluorobutyl)carbamoyl]indol-3-ylmethyl]-N-(2-methylphenylsulfonyl)benzamide, (R)-3-[2-Methoxy-4-[N-(2-methylphenylsulfonyl)carbamyl]benzyl]-1-methyl-N-(4,4,4-trifluoro-2-methylbutyl)indole-5-carboxamide, (+)-4(S)-(4-Carboxyphenylthio)-7-[4-(4-phenoxybutoxy)phenyl]-5(Z)-heptenoic acid and the compounds claimed in the PCT patent application number PCT/EP03/12581.

The words "treatment" and "treating" are to be understood as embracing treatment and/or amelioration of symptoms of a disease or condition as well as treatment of the cause of the disease or condition. Reference to "prevention" of a disease embraces prophylaxis and/or inhibition of the disease.

The aclidinium for use according to the invention may be administered by any suitable route to provide local antimuscarinic action. It is preferably administered by inhalation, e.g., as a powder, spray, or aerosol, preferably as a dry powder. Pharmaceutical compositions comprising aclidinium may be prepared using conventional diluents or excipients and techniques known in the galenic art. For example, dry powder formulations may contain a powder mix for inhalation comprising the aclidinium and a suitable powder base (carrier substance) such as lactose or starch. Use of lactose is preferred. Suitable inhaler devices are known in the art. Dosages will vary depending on, e.g., the individual, the mode and frequency of administration, and the nature and severity of the condition to be treated. Daily dosages for a 40 kg adult human may typically for example be on the order of 100-1000 micrograms of active agent in the form of dry powder for inhalation.

### Example 1.

In vitro stability of aclidinium compared with tiotropium and ipratropium and glycolpymolate stability in human plasma.

The in vitro experiments are carried out at 36°C and at a concentration of 5 µg/ml (6 µl of a 1 mg/ml dimethyl sulfoxide solution of each substance is added to a final volume of 1.2 ml). After 3 minutes of pre-incubation, reaction is started by addition of the test substances. At pre-defined times of 0, 5, 15, 30 and 60 min., aliquots of 100 µl of the plasma are separated and the reaction stopped by the addition of 1 ml of a 20 mM, pH 4.0 sodium acetate buffer solution. The test substances are replaced with buffer for the control reactions. Human plasma is obtained from volunteers by written informed consent. The blood is collected in tubes containing lithium heparin as anticoagulant, immediately centrifuged at 4°C and the resultant plasma stored at -20°C when not in use.

The determination of aclidinium, tiotropium, ipratropium and glycolpyrrolate in human plasma (100 µl) is carried out by high-performance liquid chromatography (HPLC) using UV detection, at 238 mn for aclidinium and tiotropium, and 203 nm for Ipratropium, and an automated online solid-phase extraction and injection procedure. A suitable chromatographic system consists of a high-pressure pump (model 322 Kontron for aclidinium and tiotropium and model 515 Waters for ipratropium), a Prospekt system (Spark Holland) assisted by a 233XL sampling injector (Gilson Medical Electronics), a tunable absorbance detector (model 2487, Waters Ass.), and a Digital Alpha Server 1000 4/266 computer with Acces*Chrom software (Perkin Elmer Nelson Systems, Inc.). Chromatography for aclidinium and tiotropium determination is carried out on a Spherisorb ODS2, 5 µm, 150 x 4.6 mm column (Waters Ass.) with a Guardapack µBondapak CN Precolumn (Waters Ass.) and a mobile phase (50:50 v/v for ACLIDINIUM and 22:78 v/v for tiotropium) of acetonitrile: 20 mM, pH 3.0 sodium phosphate buffer solution containing 0.2% triethylamine at a flow rate of 1 ml/min. The approximate retention times for aclidinium and tiotropium are 9.8 and 9.5 minutes respectively. Chromatography for ipratropium determination is carried out on a Symmetry C18, 5 µm, 150 x 4.6 mm column (Waters Ass.) and a mobile phase (12:88, v/v) of acetonitrile:20 mM, pH 3.0 sodium phosphate buffer solution containing 0.2% triethylamine at a flow rate of 1 ml/min. The approximate retention time of tiotropium is 9.5 minutes. The extraction of aclidinium, tiotropium and ipratropium from plasma is performed on C2 cartridges (Baker) activated with 1.5 ml of acetonitrile and conditioned with 1.5 ml of water. Plasma samples, previously diluted with 1 ml of a 20 mM, pH 4.0 sodium acetate buffer solution, were loaded into the C2 cartridges. After washing out the cartridges with 1 ml of water and 1 ml of acetonitrile:water (40:60, v/v) for aclidinium, or 3 ml of water for tiotropium, or 1 ml of water and 1 ml of acetonitrile:water (10:90, v/v) for ipratropium, the remaining components are eluted with the mobile phase over 1 minute. There are no significant endogenous peaks at retention times of the analytes that would interfere with their quantitation. The recovery of aclidinium is about 95% from human plasma. The recovery of tiotropium and ipratropium from plasma is between 80-100%. Glycopyrrolate stability in human plasma is using essentially the same procedures as for the other three drugs. The lower limit of quantitation is established at 5 ng/ml for all analytes.

Aclidinium is rapidly hydrolyzed in human plasma in its alcohol and acid metabolites. Both metabolites of aclidinium are assayed on binding for M1, M2, M3, and M4 human muscarinic receptors and are devoid of significant affinity for these receptors. The plasma half life of aclidinium in plasma is lower than 5 minutes for human. Moreover, aclidinium is stable in acid aqueous solutions (pH s 4) and the hydrolytic cleavage of the ester bond takes place at neutral and basic pHs.

In contrast, the other three antimuscarinic esters are quite resistant to degradation by esterases in plasma. Plasma degradation for tiotropium (16%), ipratropium (0%), glycolpyrrolate (9%) is not biologically significant during the time of this study (60 min).

### Example 2

Clinical Phase I study: Aclidinium bromide is tested in a Phase I, double-blind, partial cross-over, placebo controlled study to assess the activity, pharmacokinetics and tolerability of aclidinium.

Methods: 12 healthy male volunteers are randomly assigned to 1 of 4 treatment sequences comprising single doses of aclidinium (50, 300 and 600 micrograms) or placebo administered by dry powder inhaler. The washout period between administrations is at least 6 days. Efficacy endpoints are specific airway conductance (sGaw), airway resistance (Raw) and bronchial hyperresponsiveness (PC35 sGaw methacholine).

Results: Aclidinium significantly increases sGaw at all timepoints (1-24 h, p<0.001 vs placebo). Correspondingly, Raw is significantly decreased by aclidinium at all timepoints except 1 h and 24 h (p0.001 vs placebo). Aclidinium 300 and 600 micrograms also significantly reduces PC35 sGaw methacholine at all post-administration timepoints (p<0.001 vs placebo): the methacholine doses required to decrease sGaw by 235% were 142.7 and 181.7 vs 27.1 mg/mL, for aclidinium 300 and 600 micrograms vs placebo, respectively, at 24 h; and 207.1 and 256.0 vs 35.5 mg/mL, respectively, at 2 h. Neither aclidinium nor its metabolites are detected in plasma and no study-drug-related adverse events are reported.

Conclusion: Aclidinium produces significant and long-lasting protection against methacholine-induced bronchoconstriction in healthy male volunteers, demonstrating its suitability for once-daily dosing, notwithstanding that plasma levels are not even detectable.

### Example 3

Clinical Phase II study: A double-blind, randomised, placebo-controlled, cross-over trial assesses the pharmacodynamics, pharmacokinetics and tolerability of aclidinium and its effects in COPD patients

Methods: Men with COPD (FEVI <65% predicted) with demonstrated airway reversibility to ipratropium are randomised to 1 of 4 treatment sequences comprising single doses of aclidinium (100, 300 and 900 micrograms) and placebo administered by dry powder inhaler with a washout period of 1 week between doses. Lung function measurements include FEVI and FVC.

Results: 17 males (mean age 63.5 y, mean FEV, 1.63 L) participate in the study. Aclidinium (100, 300 and 900 micrograms) significantly increases mean FEVi Auk(0-24)/24 compared with placebo (1.800 [p=0.002], 1.798 [p<0.0001] and 1.827 [p0.0001] L vs 1.597 L, respectively). The increase in FEVI are statistically significant at 24 h for all doses. Aclidinium 300 and 900 micrograms produces greater peak FEV1 effects and the time to maximal onset occurres earlier than with the 100 micrograms dose. Similar trends are seen with FVC. No plasma levels of aclidinium or its alcohol metabolite are detected; low levels of its acid metabolite can be detected following the 900 microgram dose. Aclidinium is well tolerated: only 6 cases of mild or moderate headache (vs 2 with placebo) and 1 of mildly increased sweating appear possibly related to treatment.

Conclusion: Single doses of aclidinium (100, 300 and 900 micrograms) produce a rapid and long-lasting bronchodilation in patients with COPD, notwithstanding that plasma levels are not detectable.

## Claims

1. Aclidinium, for use in the treatment or prevention of a respiratory disease or condition in a patient by inhalation, which patient is suffering from or susceptible to a condition which may be exacerbated by systemic antimuscarinic activity.

2. Aclidinium, for use according to claim 1, wherein the respiratory disease or condition is selected from acute or chronic bronchitis, emphysema, asthma and chronic obstructive pulmonary disease, especially asthma and chronic obstructive pulmonary disease.

3. Aclidinium, for use according to any of the preceding claims wherein the patient is suffering from or susceptible to one or more selected from
i. schizophrenia, impaired concentration, confusion, agitation, delirium, attention deficit, impaired memory, respiratory depression,
ii. glaucoma, dry eye, increased pupil size, blurred vision, increased intraocular pressure,
iii. enlarged or obstructed prostate, difficulty urinating,
iv. narrowing or obstruction of the small intestine, enlarged colon, chronic constipation, enlarged lower esophagus, decreased gastric motility, constipation,
v. dry mouth, throat irritation, impaired sweating,
vi. cardiovascular disease (including any of restenosis, arteriosclerosis, prior stroke or heart attack, congestive heart failure), arrhythmia, tachycardia,
vii. Parkinson's Disease, Alzheimer's Disease, dementia, and
viii. myasthenia gravis

4. Aclidinium, for use according to any of the preceding claims, wherein:
the patient is a male; and/or
the patient is over sixty years old; and/or
the patient intends to drive or operate machinery during the course of treatment; and/or
the patient is receiving a second drug which is a systemically active anticholinergic agent, or an agent which may cause or exacerbate any of the conditions defined in claim 3, which second drug is preferably selected from atypical antipsychotics, tricyclic antidepressants, and antihistamines.

5. Aclidinium, for use according to claim 4, wherein the patient is receiving a drug which is intended to enhance acetylcholine function.

6. Aclidinium, for use according to any of the preceding claims, wherein the aclidinium is in the form of aclidinium bromide.

7. Aclidinium, for use according to any of the preceding claims wherein the aclidinium is in the form of a dry powder suitable for inhalation.

8. Aclidinium, for use according to any of the preceding claims, wherein the patient receives one or more additional medications for treatment of the respiratory disease or condition.

9. Aclidinium, for use according to claim 8, wherein the additional medication for treatment of the respiratory disease or condition is selected from beta-adrenergic agonists, corticosteroids or glucocorticoids, PDE IV inhibitors, antihistamines, anti-IgE antibodies, leukotriene D4 inhibitors, inhibitors of egfr-kinase, p38 kinase inhibitors and/or NK1-receptor antagonists.

10. Aclidinium, for use according to claim 9, wherein the additional medication is selected from corticosteroids and/or beta-adrenergic agonists.

11. Use of aclidinium, as defined in any one of claims 1, 6 and 7, in the manufacture of a medicament, for use in the treatment or prevention, by inhalation, of a respiratory disease or condition as defined in claim 1 or 2, in a patient as defined in any one of claims 1, 3 to 5 and 8 to 10.

12. A pharmaceutical composition, for use in the treatment or prevention, by inhalation, of a respiratory disease or condition as defined in claim 1 or 2, in a patient as defined in any one of claims 1, 3 to 5 and 8 to 10, which pharmaceutical composition comprises a pharmaceutically acceptable carrier and, as active principle, aclidinium, as defined in any one of claims 1, 6 sand 7.

13. A pharmaceutical composition for use according to claim 12, wherein the pharamceuticall acceptable carrier is selected from mono-, di- or polysaccharide and sugar alcohols, preferably lactose

## Patentansprüche

1. Aclidinium zur Verwendung bei der Behandlung oder Prävention einer respiratorischen Krankheit oder eines respiratorischen Zustandes bei einem Patienten durch Inhalation, wobei der Patient an einem Zustand, der durch systemische antimuskarinische Aktivität verschlimmert werden kann, leidet oder dafür anfällig ist.

2. Aclidinium zur Verwendung gemäß Anspruch 1, wobei die respiratorische Krankheit oder der respiratorische Zustand aus akuter oder chronischer Bronchitis, Emphysem, Asthma und chronisch-obstruktiver Lungenkrankheit, speziell Asthma und chronisch-obstruktiver Lungenkrankheit, ausgewählt ist.

3. Aclidinium zur Verwendung gemäß einem der vorangehenden Ansprüche, wobei der Patient an einer/einem oder mehreren, ausgewählt aus
i. Schizophrenie, beeinträchtigter Konzentration, Verwirrtheit, Agitation, Aufmerksamkeitsdefizit, gestörtem Erinnerungsvermögen, Atemdepression,
ii. Glaukom, trockenem Auge, vergrößerter Pupille, Sehtrübung, erhöhtem intraokulärem Druck,
iii. vergrößerter oder obstruierter Prostata, Miktionsstörung,
iv. Verengung oder Obstruktion des Dünndarms, vergrößertem Colon, chronischer Obstipation, vergrößertem unteren Ösophagus, herabgesetzter Magenmotilität, Obstipation,
v. trockenem Mund, Irritationen des Rachens, gestörtem Schwitzen,
vi. kardiovaskulärer Erkrankung (einschließlich einer von Restenose, Arteriosklerose, vor Schlaganfall oder Herzattacke, kongestivem Herzversagen), Arrhythmie, Tachykardie,
vii. Parkinsonscher Krankheit, Alzheimerscher Krankheit, Demenz und
viii. Myasthenia gravis,
leidet oder dafür anfällig ist.

4. Aclidinium zur Verwendung gemäß einem der vorangehenden Ansprüche, wobei:
der Patient ein Mann ist und/oder
der Patient über sechzig Jahre alt ist und/oder
der Patient im Verlauf der Behandlung Auto fahren möchte oder Maschinen bedienen möchte und/oder
der Patient ein zweites Arzneimittel, das ein systemisch aktives anticholinerges Mittel ist oder ein Mittel, das einen der in Anspruch 3 definierten Zustände verursachen oder verschlimmern kann, einnimmt, wobei das zweite Arzneimittel vorzugsweise aus atypischen Antipsychotika, trizyklischen Antidepressiva und Antihistaminen ausgewählt ist.

5. Aclidinium zur Verwendung gemäß Anspruch 4, wobei der Patient ein Arzneimittel einnimmt, das dazu bestimmt ist, die Acetylcholinfunktion zu erhöhen.

6. Aclidinium zur Verwendung gemäß einem der vorangehenden Ansprüche, wobei das Aclidinium in der Form von Aclidiniumbromid ist.

7. Aclidinium zur Verwendung gemäß einem der vorangehenden Ansprüche, wobei das Aclidinium in der Form eines trockenen Pulvers ist, das zur Inhalation geeignet ist.

8. Aclidinium zur Verwendung gemäß einem der vorangehenden Ansprüche, wobei der Patient eine oder mehrere zusätzliche Medikationen zur Behandlung der respiratorischen Krankheit oder des respiratorischen Zustands erhält.

9. Aclidinium zur Verwendung gemäß Anspruch 8, wobei die zusätzliche Medikation zur Behandlung der respiratorischen Krankheit oder des respiratorischen Zustandes aus betaadrenergen Agonisten, Corticosteroiden oder Glucocorticoiden, PDE-IV-Inhibitoren, Antihistaminen, Anti-IgE-Antikörpern, Leukotrien-D4-Inhibitoren, Inhibitoren von egfr-Kinase, p38-Kinase-Inhibitoren und/oder NK1-Rezeptor-Antagonisten ausgewählt ist.

10. Aclidinium zur Verwendung gemäß Anspruch 9, wobei die zusätzliche Medikation aus Corticosteroiden und/oder betaadrenergen Agonisten ausgewählt ist.

11. Verwendung von Aclidinium, wie es in einem der Ansprüche 1, 6 und 7 definiert ist, bei der Herstellung eines Medikaments zur Verwendung bei der Behandlung oder Prävention einer respiratorischen Krankheit oder eines respiratorischen Zustandes, wie in Anspruch 1 oder 2 definiert, durch Inhalation bei einem Patienten, wie in einem der Ansprüche 1, 3 bis 5 und 8 bis 10 definiert.

12. Pharmazeutische Zusammensetzung zur Verwendung bei der Behandlung oder Prävention einer respiratorischen Krankheit oder eines respiratorischen Zustandes, wie in Anspruch 1 oder 2 definiert, bei einem Patienten, wie in einem der Ansprüche 1, 3 bis 5 und 8 bis 10 definiert, durch Inhalation, wobei die pharmazeutische Zusammensetzung einen pharmazeutisch verträglichen Träger und als aktiven Wirkstoff Aclidinium, wie in einem der Ansprüche 1, 6 und 7 definiert, umfasst.

13. Pharmazeutische Zusammensetzung zur Verwendung gemäß Anspruch 12, wobei der pharmazeutisch verträgliche Träger aus Mono-, Di- oder Polysaccharid und Zuckeralkoholen ausgewählt ist, vorzugsweise Laktose ist.

## Revendications

1. Aclidinium, pour utilisation dans le traitement ou la prévention d'une maladie respiratoire ou d'un état chez un patient par inhalation, lequel patient souffre ou est sensible à un état qui peut être exacerbé par une activité antimuscarinique systémique.

2. Aclidinium, pour utilisation selon la revendication 1, dans laquelle la maladie respiratoire ou l'état est choisi parmi une bronchite aiguë ou chronique, l'emphysème, l'asthme et une maladie pulmonaire obstructive chronique, en particulier l'asthme et une maladie pulmonaire obstructive chronique.

3. Aclidinium, pour utilisation selon l'une quelconque des revendications précédentes dans lesquelles le patient souffre ou est sensible à une ou plusieurs affections parmi
i. schizophrénie, troubles de la concentration, confusion, agitation, délire, déficit d'attention, troubles de la mémoire, dépression respiratoire,
ii. glaucome, kératoconjonctivite sèche, augmentation de la taille de la pupille, vision floue, augmentation de la pression intraoculaire,
iii. augmentation de la taille et obstruction de la prostate, difficulté d'uriner,
iv. rétrécissement ou obstruction de l'intestin grêle, élargissement du côlon, constipation chronique, élargissement de l'oesophage inférieur, réduction de la motilité gastrique, constipation,
v. sécheresse buccale, irritation de la gorge, troubles des la sudation,
vi. maladie cardiovasculaire (y compris l'une quelconque parmi resténose, artériosclérose, AVC primaire ou crise cardiaque, insuffisance cardiaque congestive), arythmie, tachycardie,
vii. maladie de Parkinson, maladie d'Alzheimer, démence, et
viii. myasthénie grave

4. Aclidinium, pour utilisation selon l'une quelconque des revendications précédentes, dans laquelle :
le patient est de sexe masculin ; et/ou
le patient a plus de soixante ans ; et/ou
le patient a l'intention de conduire ou d'utiliser une machine pendant la durée du traitement ; et/ou
on administre au patient un second médicament qui est un agent anticholinergique systémiquement actif, ou
un agent qui peut provoquer ou exacerber un quelconque des états définis dans la revendication 3, lequel second médicament est de préférence choisi parmi des antipsychotiques atypiques, antidépresseurs tricycliques, et antihistaminiques.

5. Aclidinium, pour utilisation selon la revendication 4, dans laquelle on administre au patient un médicament qui a pour but de stimuler la fonction acétylcholine.

6. Aclidinium, pour utilisation selon l'une quelconque des revendications précédentes, dans laquelle l'aclidinium est sous la forme de bromure d'aclidinium.

7. Aclidinium, pour utilisation selon l'une quelconque des revendications précédentes, dans laquelle l'aclidinium est sous forme de poudre sèche convenant pour l'inhalation.

8. Aclidinium, pour utilisation selon l'une quelconque des revendications précédentes, dans laquelle on administre au patient un ou plusieurs médicaments supplémentaires pour le traitement de la maladie respiratoire ou de l'état.

9. Aclidinium, pour utilisation selon la revendication 8, dans laquelle le médicament supplémentaire pour le traitement de la maladie respiratoire ou de l'état est choisi parmi des agonistes bêta-adrénergiques, des corticostéroïdes ou des glucocorticoïdes, des inhibiteurs de PDE IV, antihistaminiques, anticorps anti-IgE, inhibiteurs de leucotriènes D4, inhibiteurs d'egfr-kinase, inhibiteurs de p38-kinase et/ou antagonistes des récepteurs NK1.

10. Aclidinium, pour utilisation selon la revendication 9, dans laquelle le médicament supplémentaire est choisi parmi des corticostéroïdes et/ou des agonistes bêta-adrénergiques.

11. Utilisation d'aclidinium, comme défini dans l'une quelconque des revendications 1, 6 et 7, dans la préparation d'un médicament, pour utilisation dans le traitement ou la prévention, par inhalation, d'une maladie respiratoire ou d'un état tel que défini dans la revendication 1 ou 2, chez un patient tel que défini dans l'une quelconque des revendications 1, 3 à 5 et 8 à 10.

12. Composition pharmaceutique, pour utilisation dans le traitement ou la prévention, par inhalation, d'une maladie respiratoire ou d'un état tel que défini dans la revendication 1 ou 2, chez un patient tel que défini dans l'une quelconque des revendications 1, 3 à 5 et 8 à 10, laquelle composition pharmaceutique comprend un véhicule pharmaceutiquement acceptable et, comme principe actif, de l'aclidinium, comme défini dans l'une quelconque des revendications 1, 6 et 7.

13. Composition pharmaceutique pour utilisation selon la revendication 12, dans laquelle le véhicule pharmaceutiquement acceptable est choisi parmi un mono-, di- ou polysaccharide et des polyols, de préférence le lactose.
